# EUROPEAN PATENT APPLICATION

(11) **EP 4 726 033 A1**
(43) Date of publication of application: **15.04.2026**
(21) Application number: 24826213.1
(22) Date of filing: 17.06.2024
(51) Int. Cl.: C12N 9/04, C12N 15/70, C12P 7/625

(54) **NOVEL ACETOACETYL-COA REDUCTASE VARIANT AND USE THEREOF**

(30) Priority: 19.06.2023 KR 20230078440
(71) Applicant: CJ Cheiljedang Corporation, Seoul 04560 (KR)
(72) Inventor: CHOI, Su Jin, Seoul 04560 (KR); SEOK, Jooyeon, Seoul 04560 (KR); LEE, Imsang, Seoul 04560 (KR); LEE, Ji Sun, Seoul 04560 (KR)
(74) Representative: Meissner Bolte Partnerschaft mbB
(86) International application number: PCT/KR2024/008345
(87) International publication number: WO 2024/262896

(57) **Abstract**

Provide are a novel protein having acetoacetyl-CoA reductase activity; a polynucleotide encoding the protein; a microorganism, which comprises the protein, the polynucleotide encoding the protein, or a vector comprising the polynucleotide; and a method of producing polyhydroxyalkanoate (PHA), comprising a step of culturing the microorganism in a medium.

## Description

### [Technical Field]

The present disclosure relates to a novel protein having acetoacetyl-CoA reductase activity; a polynucleotide encoding the protein; a microorganism, which comprises the protein, the polynucleotide encoding the protein, or a vector comprising the polynucleotide; and a method of producing polyhydroxyalkanoate (PHA), comprising a step of culturing the microorganism in a medium.

### [Background Art]

Polyhydroxyalkanoate (PHA), which has recently received attention as a biodegradable polymeric material, is a natural polymeric material having a polyester-based structure which is accumulated as a storage material for carbon sources and energy when microorganisms are deficient in nutrients such as nitrogen, phosphorus, etc. in the presence of excess carbon source.

Poly-3-hydroxybutyrate (P3HB), a representative well-known polyhydroxyalkanoate, is a material polymerized using a 3-hydroxybutylate (3HB) monomer, and has mechanical properties similar to polypropylene which is a commercially available petroleum synthetic polymer. Since P3HB is completely decomposed by microorganisms in the natural world, it is attracting attention as an environmentally friendly plastic raw material.

In this regard, the 3-hydroxybutyrate homopolymer (poly(3-hydroxybutyrate), P(3HB)), which is most widely studied in microorganisms among polymers of the polyhydroxyalkanoate family, has a high degree of crystallinity, making it brittle and hard. Since it decomposes near its melting point, there are limitations in its processability. Accordingly, research is conducted to produce the PHA copolymers by blending with new materials or copolymerizing with monomers.

Among them, a copolymer (P(3HB-co-4HB)) of 3-hydroxybutyrate and 4-hydroxybutyrate exhibits soft properties depending on the content of 4-hydroxybutyrate. In other words, P(3HB-co-4HB) is highly valued as a useful polymeric material that is applicable to a variety of products, because its physical properties may vary widely from hard crystalline plastic to highly elastic rubber depending on the ratio of monomers.

Accordingly, for the broad portfolio of PHA, further research is still needed to produce 3-hydroxybutyrate-4-hydroxybutyrate copolymers with various ratios of monomers.

### [Disclosure]

### [Technical Problem]

The present disclosure relates to a protein variant having acetoacetyl-CoA reductase activity and use thereof in producing polyhydroxyalkanoate (PHA).

### [Technical Solution]

An object of the present disclosure is to provide a protein having acetoacetyl-CoA reductase activity, in which an amino acid corresponding to position 35 of an amino acid sequence of SEQ ID NO: 1 or an amino acid sequence having 55% or more sequence identity to the amino acid sequence of SEQ ID NO: 1 is substituted with a different amino acid.

Another object of the present disclosure is to provide a polynucleotide encoding the protein.

Still another object of the present disclosure is to provide a microorganism comprising the protein, the polynucleotide encoding the protein, or a vector comprising the polynucleotide.

Still another object of the present disclosure is to provide a method of producing polyhydroxyalkanoate (PHA), the method comprising the step of culturing the microorganism in a medium.

Still another object of the present disclosure is to provide a composition for producing polyhydroxyalkanoate (PHA), the composition comprising the protein; the polynucleotide encoding the protein; or the microorganism comprising the protein, the polynucleotide encoding the protein, or the vector comprising the polynucleotide; a culture of the microorganism; or a combination of two or more thereof.

### [Advantageous Effects]

When a microorganism comprising a protein having acetoacetyl-CoA reductase activity of the present disclosure is cultured, high-yield production of polyhydroxyalkanoate (PHA) is possible, as compared to microorganisms with the existing unmodified polypeptides. Further, the variant of the present disclosure may be used in producing a 3-hydroxybutyrate-4-hydroxybutyrate copolymer with a high content of 3-hydroxybutyrate.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a protein having acetoacetyl-CoA reductase activity, in which an amino acid corresponding to position 35 of an amino acid sequence of SEQ ID NO: 1 or an amino acid sequence having 55% or more sequence identity to the amino acid sequence of SEQ ID NO: 1 is substituted with a different amino acid.

Specifically, the protein may have the amino acid sequence of SEQ ID NO: 1 or an amino acid sequence having 55% or more and less than 100% sequence identity to the amino acid sequence of SEQ ID NO: 1.

Specifically, the protein may be a protein, in which an amino acid corresponding to position 35 of any one amino acid sequence of SEQ ID NO: 50, SEQ ID NO: 51, and SEQ ID NO: 54 to SEQ ID NO: 56 is substituted with a different amino acid,
an amino acid corresponding to position 34 of an amino acid sequence of SEQ ID NO: 52 is substituted with a different amino acid, or
an amino acid corresponding to position 36 of an amino acid sequence of SEQ ID NO: 53 is substituted with a different amino acid, but is not limited thereto.

In the present disclosure, the "protein having acetoacetyl-CoA reductase activity, in which the amino acid corresponding to position 35 of the amino acid sequence of SEQ ID NO: 1 is substituted with a different amino acid", the "protein having acetoacetyl-CoA reductase activity, in which the amino acid corresponding to position 35 of any one amino acid sequence of SEQ ID NO: 50, SEQ ID NO: 51, and SEQ ID NO: 54 to SEQ ID NO: 56 is substituted with a different amino acid", the "protein having acetoacetyl-CoA reductase activity, in which the amino acid corresponding to position 34 of the amino acid sequence of SEQ ID NO: 52 is substituted with a different amino acid", or the "protein having acetoacetyl-CoA reductase activity, in which the amino acid corresponding to position 36 of the amino acid sequence of SEQ ID NO: 53 is substituted with a different amino acid" may refer to an acetoacetyl-CoA reductase variant comprising one or more amino acid substitutions in an amino acid sequence of an unmodified parent acetoacetyl-CoA reductase having acetoacetyl-CoA reductase activity, and may be used interchangeably with the term "acetoacetyl-CoA reductase variant", "variant", or "variant polypeptide", etc.

As used herein, the term "acetoacetyl-CoA reductase" refers to an enzyme that converts acetoacetyl-CoA into 3-hydroxybutyryl-CoA.

In the present disclosure, the "parent acetoacetyl-CoA reductase" refers to acetoacetyl-CoA reductase to which modification is applied in order to produce the acetoacetyl-CoA reductase variant or variant polypeptide of the present disclosure. Specifically, the parent acetoacetyl-CoA reductase or parent sequence may be a naturally occurring polypeptide or a wild-type polypeptide, a mature polypeptide thereof, and may comprise a variant or functional fragment thereof, but is not limited thereto, as long as it is a polypeptide which may be a parent of the variant with the acetoacetyl-CoA reductase activity.

In the present disclosure, the parent acetoacetyl-CoA reductase may be a protein having acetoacetyl-CoA reductase activity, which is encoded by *phaB* gene, but is not particularly limited to its type, as long as it has the activity corresponding to that of acetoacetyl-CoA reductase and capable of increasing polyhydroxyalkanoate (PHA)-producing ability by enhancing the activity in microorganisms.

Specifically, examples of the parent acetoacetyl-CoA reductase protein may comprise any one amino acid sequence of SEQ ID NO: 1 and SEQ ID NO: 50 to SEQ ID NO: 5, or an amino acid sequence having 55% or more homology or identity thereto, but are not limited thereto, as long as they have the acetoacetyl-CoA reductase activity. Specifically, the amino acid sequence may comprise any one amino acid sequence of SEQ ID NO: 1 and SEQ ID NO: 50 to SEQ ID NO: 56; or an amino acid sequence having at least 55%, 60%, 70%, 80%, 85%, 90%, 95%, 96%, 97%, 98%, or 99% or more homology or identity to any one amino acid sequence of SEQ ID NO: 1 and SEQ ID NO: 50 to SEQ ID NO: 56. It is also apparent that an accessory protein having an amino acid sequence with deletion, modification, substitution, or addition of some sequence also falls within the scope of the present disclosure as long as the amino acid sequence has such homology or identity and exhibits efficacy corresponding to that of the protein. Any one amino acid sequence of SEQ ID NO: 1 and SEQ ID NO: 50 to SEQ ID NO: 56 may be obtained from the NCBI's GenBank or Kyoto Encyclopedia of Genes and Genomes (KEGG), which is a known database.

Specifically, any one amino acid sequence of SEQ ID NO: 50 to SEQ ID NO: 56 has 55% or more and less than 100% sequence identity to the amino acid sequence of SEQ ID NO: 1.

In the present disclosure, the acetoacetyl-CoA reductase may be acetoacetyl-CoA reductase derived from *Cupriavidus necator* (*Alcaligenes eutrophus, Ralstonia eutropha, Alcaligenes eutrophus* H16), *Pseudomonas putida* (*P. putida*), *Alcaligenes latus (Azohydromonas lata, Azohydromonas australica), Allochromatium vinosum* (*Allochromatium vinosum* DSM 180), *Azotobacter Beijerinckii, Pandoraea* sp. (*Pandoraea* sp. B-6), *Burkholderiaceae bacterium* (*Burkholderiaceae bacterium* 16)*,* or *Candidatus Accumulibacter phosphatis,* but is not limited thereto.

The acetoacetyl-CoA reductase derived from *Cupriavidus necator* (*Alcaligenes eutrophus, Ralstonia eutropha, Alcaligenes eutrophus* H16), which is presented as an example in the present disclosure, may be a polypeptide/protein comprising the amino acid sequence represented by SEQ ID NO: 1, but is not limited thereto.

The acetoacetyl-CoA reductase derived from *Pseudomonas putida* (*P. putida*), which is presented as an example in the present disclosure, may be a polypeptide/protein comprising the amino acid sequence represented by SEQ ID NO: 50, but is not limited thereto.

The acetoacetyl-CoA reductase derived from *Alcaligenes latus* (*Azohydromonas lata, Azohydromonas australica*), which is presented as an example in the present disclosure, may be a polypeptide/protein comprising the amino acid sequence represented by SEQ ID NO: 51, but is not limited thereto.

The acetoacetyl-CoA reductase derived from *Alcaligenes latus Allochromatium vinosum* (*Allochromatium vinosum* DSM 180), which is presented as an example in the present disclosure, may be a polypeptide/protein comprising the amino acid sequence represented by SEQ ID NO: 52, but is not limited thereto.

The acetoacetyl-CoA reductase derived from *Azotobacter Beijerinckii,* which is presented as an example in the present disclosure, may be a polypeptide/protein comprising the amino acid sequence represented by SEQ ID NO: 53, but is not limited thereto.

The acetoacetyl-CoA reductase derived from *Pandoraea* sp. (*Pandoraea* sp. B-6), which is presented as an example in the present disclosure, may be a polypeptide/protein comprising the amino acid sequence represented by SEQ ID NO: 54, but is not limited thereto.

The acetoacetyl-CoA reductase derived from *Burkholderiaceae bacterium* (*Burkholderiaceae bacterium* 16), which is presented as an example in the present disclosure, may be a polypeptide/protein comprising the amino acid sequence represented by SEQ ID NO: 55, but is not limited thereto.

The acetoacetyl-CoA reductase derived from *Candidatus Accumulibacter phosphatis,* which is presented as an example in the present disclosure, may be a polypeptide/protein comprising the amino acid sequence represented by SEQ ID NO: 56, but is not limited thereto.

Further, the polynucleotide sequence encoding the parent acetoacetyl-CoA reductase may be a polynucleotide sequence encoding a protein that exhibits the acetoacetyl-CoA reductase activity and improves polyhydroxyalkanoate (PHA)-producing ability by enhancing the activity in microorganisms, for example, a polynucleotide sequence encoding the acetoacetyl-CoA reductase (SEQ ID NO: 1) derived from *Cupriavidus necator* (*Alcaligenes eutrophus, Ralstonia eutropha, Alcaligenes eutrophus* H16), the acetoacetyl-CoA reductase (SEQ ID NO: 50) derived from *Pseudomonas putida* (*P. putida*), the acetoacetyl-CoA reductase (SEQ ID NO: 51) derived from *Alcaligenes latus* (*Azohydromonas lata, Azohydromonas australica*), the acetoacetyl-CoA reductase (SEQ ID NO: 52) derived from *Allochromatium vinosum* (*Allochromatium vinosum* DSM 180), the acetoacetyl-CoA reductase (SEQ ID NO: 53) derived from *Azotobacter Beijerinckii,* the acetoacetyl-CoA reductase (SEQ ID NO: 54) derived from *Pandoraea* sp. (*Pandoraea* sp. B-6), the acetoacetyl-CoA reductase (SEQ ID NO: 55) derived from *Burkholderiaceae bacterium* (*Burkholderiaceae bacterium* 16), or the acetoacetyl-CoA reductase (SEQ ID NO: 56) derived from *Candidatus Accumulibacter phosphatis,* but is not limited thereto. For example, it may be encoded by a polynucleotide having or comprising a nucleotide sequence of SEQ ID NO: 2, a nucleotide sequence of SEQ ID NO: 64, a nucleotide sequence of SEQ ID NO: 65, a nucleotide sequence of SEQ ID NO: 66, a nucleotide sequence of SEQ ID NO: 67, a nucleotide sequence of SEQ ID NO: 68, a nucleotide sequence of SEQ ID NO: 69, or a nucleotide sequence of SEQ ID NO: 70; or a nucleotide sequence having 55% or more, 60% or more, 70% or more, 75% or more, 80% or more, 85% or more, 90% or more, 95% or more, 96% or more, 97% or more, 98% or more or 99% or more homology or identity to the nucleotide sequence of SEQ ID NO: 2, the nucleotide sequence of SEQ ID NO: 64, the nucleotide sequence of SEQ ID NO: 65, the nucleotide sequence of SEQ ID NO: 66, the nucleotide sequence of SEQ ID NO: 67, the nucleotide sequence of SEQ ID NO: 68, the nucleotide sequence of SEQ ID NO: 69, or the nucleotide sequence of SEQ ID NO: 70, or a polynucleotide consisting of or essentially consisting of the nucleotide sequence of SEQ ID NO: 2, the nucleotide sequence of SEQ ID NO: 64, the nucleotide sequence of SEQ ID NO: 65, the nucleotide sequence of SEQ ID NO: 66, the nucleotide sequence of SEQ ID NO: 67, the nucleotide sequence of SEQ ID NO: 68, the nucleotide sequence of SEQ ID NO: 69, or the nucleotide sequence of SEQ ID NO: 70 or a nucleotide sequence having 55% or more, 60% or more, 70% or more, 75% or more, 80% or more, 85% or more, 90% or more, 95% or more, 96% or more, 97% or more, 98% or more or 99% or more homology or identity to the nucleotide sequence of SEQ ID NO: 2, the nucleotide sequence of SEQ ID NO: 64, the nucleotide sequence of SEQ ID NO: 65, the nucleotide sequence of SEQ ID NO: 66, the nucleotide sequence of SEQ ID NO: 67, the nucleotide sequence of SEQ ID NO: 68, the nucleotide sequence of SEQ ID NO: 69, or the nucleotide sequence of SEQ ID NO: 70, but is not limited thereto. The nucleotide sequence may undergo modifications in the coding region due to codon degeneracy, or may also undergo various modifications in the coding region without changing the amino acid sequence in consideration of the codons preferred in an organism in which the nucleotide sequence is to be expressed. It is also apparent that a polynucleotide sequence having deletion, modification, substitution, or addition of some sequence also falls within the scope of the present disclosure as long as the polynucleotide sequence has such homology or identity and encodes a protein that substantially exhibits efficacy identical or corresponding to that of the protein.

As used herein, the term "variant" refers to a polypeptide in which one or more amino acids are conservatively substituted and/or modified so as to be different from the amino acid sequence of the variant before modification, while retaining functions or properties thereof. Such a variant may generally be identified by modifying one or more amino acids in the amino acid sequence of the polypeptide and evaluating the properties of the modified polypeptide. In other words, the ability of the variant may be increased, unchanged, or decreased, as compared to the polypeptide before modification. Further, some variants may comprise variants in which one or more portions, such as an N-terminal leader sequence or transmembrane domain, have been removed. Other variants may comprise variants in which a portion has been removed from the N- and/or C-terminus of a mature protein. The term "variant" may also be used as a modification, modified polypeptide, modified protein, mutant, mutein, divergent, etc., and any term is not limited, as long as it is used in a sense of being mutated.

The variant may also comprise deletion or addition of amino acids that have minimal influence on the properties and secondary structure of the polypeptide. For example, a polypeptide may be conjugated to a signal (or leader) sequence at the N-terminus of the protein, which co-translationally or post-translationally directs transfer of the protein. The polypeptide may also be conjugated to other sequence or a linker for identification, purification, or synthesis of the polypeptide.

As used herein, the term "acetoacetyl-CoA reductase variant", "variant", or "variant polypeptide" refers to a protein, in which one or more amino acids are different from the amino acid sequence of the parent acetoacetyl-CoA reductase, while having the acetoacetyl-CoA reductase activity.

Specifically, the acetoacetyl-CoA reductase variant of the present disclosure may be an acetoacetyl-CoA reductase variant, in which an amino acid corresponding to position 35 of any one amino acid sequence of SEQ ID NO: 1, SEQ ID NO: 50, SEQ ID NO: 51, and SEQ ID NO: 54 to SEQ ID NO: 56 is substituted with a different amino acid, or an amino acid corresponding to position 34 of an amino acid sequence of SEQ ID NO: 52 is substituted with a different amino acid, or an amino acid corresponding to position 36 of an amino acid sequence of SEQ ID NO: 53 is substituted with a different amino acid, but is not limited thereto.

The "different amino acid" is not limited as long as it is an amino acid different from the amino acid before substitution. Meanwhile, when it is expressed that "a specific amino acid has been substituted", it is obvious that the amino acid is substituted with an amino acid different from the amino acid before substitution, even though it is not specifically stated that the amino acid has been substituted with a different amino acid.

Amino acids may generally be classified based on similarity of polarity, charge, solubility, hydrophobicity, hydrophilicity, and/or amphipathic nature of residues.

Examples of this classification may comprise positively charged (basic) amino acids such as arginine, lysine, and histidine; negatively charged (acidic) amino acids such as glutamic acid and aspartic acid; amino acids with nonpolar side chains (nonpolar amino acids) such as glycine, alanine, valine, leucine, isoleucine, methionine, phenylalanine, tryptophan, and proline; amino acids with polar or hydrophilic side chains (polar amino acids) such as serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto.

For example, when it is expressed that "an amino acid corresponding to position 35 of SEQ ID NO: 1 is substituted with a different amino acid", it may mean that the amino acid is substituted with alanine, glutamate, phenylalanine, arginine, aspartate, cysteine, asparagine, glutamine, histidine, proline, serine, tyrosine, isoleucine, lysine, tryptophan, valine, methionine, threonine, or leucine, excluding glycine, but is not limited thereto.

In the present disclosure, although being described as "a protein having an amino acid sequence represented by a specific SEQ ID NO.", it is obvious that any protein having an amino acid sequence with deletion, modification, substitution, conservative substitution, or addition of some sequence may be used in the present disclosure as long as the protein has activity identical or corresponding to that of the protein consisting of the amino acid sequence of the corresponding SEQ ID NO. For example, as long as a protein has activity identical or corresponding to that of the variant protein, it does not exclude addition of a sequence, which does not alter the function of the protein, upstream or downstream of the amino acid sequence, a naturally occurring mutation, a silent mutation thereof, or conservative substitution, and it is obvious that such a sequence addition or mutation also falls within the scope of the present disclosure.

As used herein, the "position N" may comprise position N and an amino acid position corresponding to the position N. Specifically, it may comprise an amino acid position corresponding to any amino acid residue in a mature polypeptide disclosed in a particular amino acid sequence. The particular amino acid sequence may be any one amino acid sequence of SEQ ID NO: 1 and SEQ ID NO: 50 to SEQ ID NO: 56.

As used herein, the term "corresponding to" refers to an amino acid residue at a position listed in a polypeptide, or an amino acid residue that is similar, identical, or homologous to a residue listed in a polypeptide. Confirming the amino acid at the corresponding position may be determining a specific amino acid in a sequence that refers to a specific sequence. The "corresponding region" used herein generally refers to a similar or corresponding position in a related protein or reference protein.

For example, any amino acid sequence is aligned with SEQ ID NO: 1, and based on this, each amino acid residue of the amino acid sequence may be numbered by reference to the numerical position of the amino acid residue corresponding to the amino acid residue of SEQ ID NO: 1. For example, a sequence alignment algorithm such as that described herein may confirm the position of an amino acid, or a position where a modification such as substitution, insertion or deletion occurs, compared to a query sequence (also referred to as a "reference sequence").

For such alignment, for example, Needleman-Wunsch algorithm (Needleman and Wunsch, 1970, J. Mol. Biol. 48: 443-453), Needleman program of EMBOSS package (EMBOSS: The European Molecular Biology Open Software Suite, Rice et al., 2000), Trends Genet. 16: 276-277), and the like may be used without being limited thereto, and sequence alignment programs, pairwise sequence comparison algorithm, and the like known in the art may be appropriately used.

In the present disclosure, the amino acid sequence of SEQ ID NO: 52 or the amino acid sequence of SEQ ID NO: 53 is aligned with any one amino acid sequence of SEQ ID NO: 1, SEQ ID NO: 50, SEQ ID NO: 51, and SEQ ID NO: 54 to SEQ ID NO: 56, the amino acid corresponding to position 34 of the amino acid sequence of SEQ ID NO: 52 and the amino acid corresponding to position 36 of the amino acid sequence of SEQ ID NO: 53 correspond to position 35 of the amino acid residue corresponding to the amino acid residue of any one amino acid sequence of SEQ ID NO: 1, SEQ ID NO: 50, SEQ ID NO: 51, and SEQ ID NO: 54 to SEQ ID NO: 56.

In other words, through sequence alignment known in the art, those skilled in the art may recognize that the variant of the present disclosure has substitution of the amino acid corresponding to position 35 with a different amino acid when any one amino acid sequence of SEQ ID NO: 50 to SEQ ID NO: 56 is aligned with SEQ ID NO: 1, and based on this, each amino acid residue of the amino acid sequence is numbered by reference to the numerical position of the amino acid residue corresponding to the amino acid residue of SEQ ID NO: 1.

In one embodiment, the acetoacetyl-CoA reductase variant of the present disclosure may be a polypeptide in which the amino acid glycine corresponding to position 35 of SEQ ID NO: 1 is substituted with an amino acid selected from the group consisting of alanine, arginine, valine, leucine, methionine, isoleucine, threonine, asparagine, glutamine, proline, serine, tryptophan, phenylalanine, histidine, cysteine, tyrosine, lysine, aspartate, and glutamic acid, but is not limited thereto.

In any one embodiment of the above-described embodiments, the acetoacetyl-CoA reductase variant of the present disclosure may be a polypeptide in which the amino acid glycine corresponding to position 35 of SEQ ID NO: 1 is substituted with alanine, but is not limited thereto.

In one embodiment, the acetoacetyl-CoA reductase variant of the present disclosure may be a polypeptide in which the amino acid glycine corresponding to position 35 of SEQ ID NO: 50 is substituted with an amino acid selected from the group consisting of alanine, arginine, valine, leucine, methionine, isoleucine, threonine, asparagine, glutamine, proline, serine, tryptophan, phenylalanine, histidine, cysteine, tyrosine, lysine, aspartate, and glutamic acid, but is not limited thereto.

In any one embodiment of the above-described embodiments, the acetoacetyl-CoA reductase variant of the present disclosure may be a polypeptide in which the amino acid glycine corresponding to position 35 of SEQ ID NO: 50 is substituted with alanine, but is not limited thereto.

In one embodiment, the acetoacetyl-CoA reductase variant of the present disclosure may be a polypeptide in which the amino acid glycine corresponding to position 35 of SEQ ID NO: 51 is substituted with an amino acid selected from the group consisting of alanine, arginine, valine, leucine, methionine, isoleucine, threonine, asparagine, glutamine, proline, serine, tryptophan, phenylalanine, histidine, cysteine, tyrosine, lysine, aspartate, and glutamic acid, but is not limited thereto.

In any one embodiment of the above-described embodiments, the acetoacetyl-CoA reductase variant of the present disclosure may be a polypeptide in which the amino acid glycine corresponding to position 35 of SEQ ID NO: 51 is substituted with alanine, but is not limited thereto.

In one embodiment, the acetoacetyl-CoA reductase variant of the present disclosure may be a polypeptide in which the amino acid histidine corresponding to position 34 of SEQ ID NO: 52 is substituted with an amino acid selected from the group consisting of alanine, arginine, valine, leucine, methionine, isoleucine, threonine, asparagine, glutamine, proline, serine, tryptophan, phenylalanine, glycine, cysteine, tyrosine, lysine, aspartate, and glutamic acid, but is not limited thereto.

In any one embodiment of the above-described embodiments, the acetoacetyl-CoA reductase variant of the present disclosure may be a polypeptide in which the amino acid histidine corresponding to position 34 of SEQ ID NO: 52 is substituted with alanine, but is not limited thereto.

The amino acid corresponding to position 34 of the amino acid sequence of SEQ ID NO: 52 may be the amino acid corresponding to position 35 when the amino acid sequence of SEQ ID NO: 52 is aligned with SEQ ID NO: 1, and based on this, each amino acid residue of the amino acid sequence is numbered by reference to the numerical position of the amino acid residue corresponding to the amino acid residue of SEQ ID NO: 1.

In one embodiment, the acetoacetyl-CoA reductase variant of the present disclosure may be a polypeptide in which the amino acid threonine corresponding to position 36 of SEQ ID NO: 53 is substituted with an amino acid selected from the group consisting of alanine, arginine, valine, leucine, methionine, isoleucine, histidine, asparagine, glutamine, proline, serine, tryptophan, phenylalanine, glycine, cysteine, tyrosine, lysine, aspartate, and glutamic acid, but is not limited thereto.

In any one embodiment of the above-described embodiments, the acetoacetyl-CoA reductase variant of the present disclosure may be a polypeptide in which the amino acid threonine corresponding to position 36 of SEQ ID NO: 53 is substituted with alanine, but is not limited thereto.

The amino acid corresponding to position 36 of the amino acid sequence of SEQ ID NO: 53 may be the amino acid corresponding to position 35 when the amino acid sequence of SEQ ID NO: 53 is aligned with SEQ ID NO: 1, and based on this, each amino acid residue of the amino acid sequence is numbered by reference to the numerical position of the amino acid residue corresponding to the amino acid residue of SEQ ID NO: 1.

In one embodiment, the acetoacetyl-CoA reductase variant of the present disclosure may be a polypeptide in which the amino acid serine corresponding to position 35 of SEQ ID NO: 54 is substituted with an amino acid selected from the group consisting of alanine, arginine, valine, leucine, methionine, isoleucine, histidine, asparagine, glutamine, proline, threonine, tryptophan, phenylalanine, glycine, cysteine, tyrosine, lysine, aspartate, and glutamic acid, but is not limited thereto.

In any one embodiment of the above-described embodiments, the acetoacetyl-CoA reductase variant of the present disclosure may be a polypeptide in which the amino acid serine corresponding to position 35 of SEQ ID NO: 54 is substituted with alanine, but is not limited thereto.

In one embodiment, the acetoacetyl-CoA reductase variant of the present disclosure may be a polypeptide in which the amino acid threonine corresponding to position 35 of SEQ ID NO: 55 is substituted with an amino acid selected from the group consisting of alanine, arginine, valine, leucine, methionine, isoleucine, histidine, asparagine, glutamine, proline, serine, tryptophan, phenylalanine, glycine, cysteine, tyrosine, lysine, aspartate, and glutamic acid, but is not limited thereto.

In any one embodiment of the above-described embodiments, the acetoacetyl-CoA reductase variant of the present disclosure may be a polypeptide in which the amino acid threonine corresponding to position 35 of SEQ ID NO: 55 is substituted with alanine, but is not limited thereto.

In one embodiment, the acetoacetyl-CoA reductase variant of the present disclosure may be a polypeptide in which the amino acid histidine corresponding to position 35 of SEQ ID NO: 56 is substituted with an amino acid selected from the group consisting of alanine, arginine, valine, leucine, methionine, isoleucine, threonine, asparagine, glutamine, proline, serine, tryptophan, phenylalanine, glycine, cysteine, tyrosine, lysine, aspartate, and glutamic acid, but is not limited thereto.

In any one embodiment of the above-described embodiments, the acetoacetyl-CoA reductase variant of the present disclosure may be a polypeptide in which the amino acid histidine corresponding to position 35 of SEQ ID NO: 56 is substituted with alanine, but is not limited thereto.

In another embodiment, the acetoacetyl-CoA reductase variant of the present disclosure may be a protein in which the amino acid corresponding to position 35 of the amino acid sequence of SEQ ID NO: 1 is substituted with alanine, the amino acid corresponding to position 35 of any one amino acid sequence of SEQ ID NO: 50, SEQ ID NO: 51, and SEQ ID NO: 54 to SEQ ID NO: 56 is substituted with alanine, the amino acid corresponding to position 34 of the amino acid sequence of SEQ ID NO: 52 is substituted with alanine, or the amino acid corresponding to position 36 of the amino acid sequence of SEQ ID NO: 53 is substituted with alanine.

Meanwhile, through sequence alignment known in the art, those skilled in the art may recognize an amino acid in any amino acid sequence, which corresponds to position 35 of any one amino acid sequence of SEQ ID NO: 1, SEQ ID NO: 50, SEQ ID NO: 51, and SEQ ID NO: 54 to SEQ ID NO: 56 of the present disclosure, position 34 of the amino acid sequence of SEQ ID NO: 52, or position 36 of the amino acid sequence of SEQ ID NO: 53, and even though not separately described in the present disclosure, when "an amino acid at a specific position of a specific SEQ ID NO." is described, it is obvious that the amino acid comprises "an amino acid at position corresponding thereto" in any amino acid sequence.

In one embodiment, the protein having acetoacetyl-CoA reductase activity (or acetoacetyl-CoA reductase variant), in which the amino acid corresponding to position 35 of the amino acid sequence of SEQ ID NO: 1 of the present disclosure is substituted with a different amino acid, may have the amino acid sequence of SEQ ID NO: 1 or an amino acid sequence having 55% or more and less than 100% sequence identity to the amino acid sequence of SEQ ID NO: 1, but is not limited thereto.

Specifically, any one amino acid of SEQ ID NO: 50 to SEQ ID NO: 56 may have 55% or more and less than 100% sequence identity to the amino acid sequence of SEQ ID NO: 1.

Specifically, the variant of the present disclosure may comprise those in which the amino acid at position 35 from the N-terminus of any one amino acid sequence of SEQ ID NO: 1, SEQ ID NO: 50, SEQ ID NO: 51, and SEQ ID NO: 54 to SEQ ID NO: 56 is substituted with a different amino acid, the amino acid corresponding to position 34 from the N-terminus of the amino acid sequence of SEQ ID NO: 52 is substituted with a different amino acid, or the amino acid corresponding to position 36 from the N-terminus of the amino acid sequence of SEQ ID NO: 53 is substituted with a different amino acid, in the amino acid sequence of SEQ ID NO: 1 or in the amino acid sequence having at least 55%, 60%, 70%, 75%, 80%, 85%, 90%, 95%, 96%, 97%, 98%, 99% or more; and less than 100% homology or identity to the amino acid sequence of SEQ ID NO: 1. It is also apparent that a variant having an amino acid sequence having deletion, modification, substitution, conservative substitution, or addition of some sequence also falls within the scope of the present disclosure as long as the amino acid sequence has such homology or identity and exhibits efficacy corresponding to that of the variant of the present disclosure.

For example, the variant of the present disclosure may have and/or may comprise any one amino acid sequence of SEQ ID NO: 3 and SEQ ID NO: 57 to SEQ ID NO: 63, or may essentially consist of the amino acid sequence. Alternatively, the variant may comprise an amino acid sequence having at least 55%, 60%, 70%, 75%, 80%, 85%, 90%, 95%, 96%, 97%, 98%, 99%, 99.5%, 99.7% or 99.9% or more, or less than 100% homology or identity to an amino acid sequence selected from the group consisting of SEQ ID NO: 3 and SEQ ID NO: 57 to SEQ ID NO: 63.

For example, there are cases of having addition or deletion of a sequence which does not alter the function of the variant of the present disclosure at the N-terminus, C-terminus, and/or inside the amino acid sequence, a naturally occurring mutation, a silent mutation thereof, or a conservative substitution.

As used herein, the term "conservative substitution" means substituting an amino acid with another amino acid having similar structural and/or chemical properties. Such amino acid substitution may generally occur based on similarity in the polarity, charge, solubility, hydrophobicity, hydrophilicity and/or amphipathic nature of the residue. For example, positively charged (basic) amino acids comprise arginine, lysine, and histidine; negatively charged (acidic) amino acids comprise glutamic acid and aspartic acid; aromatic amino acids comprise phenylalanine, tryptophan, and tyrosine; and hydrophobic amino acids comprise alanine, valine, isoleucine, leucine, methionine, phenylalanine, tyrosine, and tryptophan. Further, amino acids may be classified into amino acids with electrically charged side chains and amino acids with uncharged side chains. The amino acids with electrically charged side chains comprise aspartic acid, glutamic acid, lysine, arginine, and histidine, and the amino acids with uncharged side chains may be further classified into nonpolar amino acids or polar amino acids. The nonpolar amino acids comprise glycine, alanine, valine, leucine, isoleucine, methionine, phenylalanine, tryptophan, and proline, and polar amino acids comprise serine, threonine, cysteine, tyrosine, asparagine, and glutamine. Commonly, the conservative substitution may have little or no effect on the activity of produced polypeptide. Commonly, the conservative substitution may have little or no effect on the activity of protein or polypeptide.

In one embodiment, the acetoacetyl-CoA reductase variant of the present disclosure may have enhanced acetoacetyl-CoA reductase activity, but is not limited thereto. Further, the variant of the present disclosure may have activity to increase the polyhydroxyalkanoate (PHA)-producing ability, as compared to the wild-type polypeptide with the acetoacetyl-CoA reductase activity, but is not limited thereto.

Another aspect of the present disclosure provides a polynucleotide encoding the variant of the present disclosure.

As used herein, the term "polynucleotide" refers to a DNA or RNA strand with a certain length or more, serving as a polymer of nucleotides in which nucleotide monomers are connected in a long chain by covalent bonds, and more specifically, refers to a polynucleotide fragment encoding the variant.

The polynucleotide encoding the acetoacetyl-CoA reductase variant of the present disclosure may comprise any polynucleotide sequence without limitation, as long as it is a polynucleotide sequence encoding the acetoacetyl-CoA reductase variant of the present disclosure. For example, the polynucleotide encoding the acetoacetyl-CoA reductase variant of the present disclosure may be a polynucleotide sequence encoding the amino acid sequence of the acetoacetyl-CoA reductase variant of the present disclosure, but is not limited thereto.

The polynucleotide of the present disclosure may undergo various modifications in the coding region without changing the amino acid sequence of the variant of the present disclosure in consideration of codon degeneracy or the codons preferred in an organism in which the variant of the present disclosure is to be expressed. Therefore, it is also apparent that a polynucleotide which may be translated into a polypeptide consisting of the amino acid sequence of the variant of the present disclosure or a polypeptide having homology or identity thereto due to codon degeneracy may also be comprised.

For example, the polynucleotide encoding the acetoacetyl-CoA reductase variant of the present disclosure may comprise, for example, a nucleotide sequence of SEQ ID NO: 71 to SEQ ID NO: 78; or a nucleotide sequence having 55%, 60%, 70%, 75%, 80%, 85%, 90%, 95%, 96%, 97%, 98%, 99%, 99.5%, 99.7% or 99.9% or more homology or identity to the nucleotide sequence of SEQ ID NO: 71 to SEQ ID NO: 78, but is not limited thereto. It is also apparent that a polynucleotide sequence having deletion, modification, substitution, conservative substitution, or addition of some sequence also falls within the scope of the present disclosure as long as the polynucleotide sequence has such homology or identity and encodes the amino acid sequence of the acetoacetyl-CoA reductase variant of the present disclosure.

Further, the polynucleotide of the present disclosure may comprise a probe that may be prepared from a known gene sequence, for example, may comprise any sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, conditions in which polynucleotides having higher homology or identity, i.e., polynucleotides having 55% or more, 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or conditions in which washing is performed once, specifically, twice to three times at a salt concentration and temperature equivalent to 60°C, 1XSSC, 0.1% SDS, specifically 60°C, 0.1XSSC, 0.1% SDS, more specifically 68°C, 0.1XSSC, 0.1% SDS, which are washing conditions for common Southern hybridization, may be listed.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition comprising a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

As used herein, the term "homology" or "identity" refers to a degree of similarity between two given amino acid sequences or nucleotide sequences, and may be expressed as a percentage. The terms homology and identity may often be used interchangeably with each other.

The sequence homology or identity of a conserved polynucleotide or polypeptide is determined by standard alignment algorithms, and the default gap penalty established by a program used may be used together. Substantially, homologous or identical sequences may generally hybridize under moderately or highly stringent conditions to the entirety or a part of the sequence. It is apparent that hybridization also comprises hybridization with a polynucleotide comprising a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package (Devereux, J., et al, Nucleic Acids Research 12: 387(1984)), BLASTP, BLASTN, FASTA(Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST of the National Center for Biotechnology Information or ClustalW may be used to determine the homology, similarity, or identity.

The homology, similarity, or identity between polynucleotides or polypeptides may be determined by comparing sequence information using the GAP computer program as introduced by, for example, Needleman et al.(1970), J Mol Biol. 48:443, as disclosed by Smith and Waterman, Adv. Appl. Math(1981) 2:482. Briefly, the GAP program defines the same as the value obtained by dividing the number of similarly aligned symbols (i.e., nucleotides or amino acids) by the total number of the symbols in the shorter of the two sequences. The default parameters for the GAP program may comprise: (1) a binary comparison matrix (comprising a value 1 for identity and a value 0 for non-identity) and the weighted comparison matrix of Gribskov et al(1986) Nucl. Acids Res. 14: 6745 as disclosed in Schwartz and Dayhoff, eds., Atlas Of Protein Sequence And Structure, National Biomedical Research Foundation, pp. 353-358(1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps. Therefore, the term "homology" or "identity", as used herein, represents relevance between sequences.

Still another aspect of the present disclosure provides a vector comprising the polynucleotide of the present disclosure. The vector may be an expression vector for expressing the polynucleotide in microorganisms, but is not limited thereto.

As used herein, the term "vector" may comprise a DNA construct comprising a nucleotide sequence of a polynucleotide encoding a desired polypeptide which is operably linked to a suitable expression regulatory region (or expression control sequence) so that the desired polypeptide may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable microorganism, and then replicate or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2, pACYC177, pACYC184, pCL, pECCG117, pUC19, pBR322, pMW118, pCC1BAC vector, etc. may be used.

For example, a polynucleotide encoding a desired polypeptide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting the cells transformed with vectors, i.e., for confirming the insertion of a desired nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a microorganism so that the polypeptide encoded by the polynucleotide may be expressed in the microorganism. The transformed polynucleotide may be located regardless of the position, either by being inserted into the chromosome of the microorganism or located outside the chromosome as long as it may be expressed in the microorganism. Further, the polynucleotide comprises DNA and/or RNA encoding a desired polypeptide. The polynucleotide may be introduced in any form as long as it may be introduced into a microorganism and expressed. For example, the polynucleotide may be introduced into a microorganism in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a microorganism in its own form and operably linked to a sequence required for expression in the microorganism, but is not limited thereto.

As used herein, the term "operably linked" means that the polynucleotide sequence is functionally linked to a promoter sequence that initiates and mediates transcription of the polynucleotide encoding the desired variant of the present disclosure.

Still another aspect of the present disclosure provides a microorganism comprising the protein, the polynucleotide encoding the protein, or the vector comprising the polynucleotide.

Specifically, provided is a microorganism comprising the protein having acetoacetyl-CoA reductase activity, in which an amino acid corresponding to position 35 of the amino acid sequence of SEQ ID NO: 1 or an amino acid sequence having 55% or more sequence identity to the amino acid sequence of SEQ ID NO: 1 is substituted with a different amino acid, the polynucleotide encoding the protein, or the vector comprising the polynucleotide.

In one embodiment, the protein may have the amino acid sequence of SEQ ID NO: 1 or an amino acid sequence having 55% or more and less than 100% sequence identity to the amino acid sequence of SEQ ID NO: 1.

In one embodiment, the protein may be a protein in which the amino acid corresponding to position 35 of any one amino acid sequence of SEQ ID NO: 50, SEQ ID NO: 51, and SEQ ID NO: 54 to SEQ ID NO: 56 is substituted with a different amino acid, the amino acid corresponding to position 34 of the amino acid sequence of SEQ ID NO: 52 is substituted with a different amino acid, or the amino acid corresponding to position 36 of the amino acid sequence of SEQ ID NO: 53 is substituted with a different amino acid.

In one embodiment, the protein may be a protein in which the amino acid corresponding to position 35 of the amino acid sequence of SEQ ID NO: 1 is substituted with alanine, or the amino acid corresponding to position 35 of any one amino acid sequence of SEQ ID NO: 50, SEQ ID NO: 51, and SEQ ID NO: 54 to SEQ ID NO: 56 is substituted with alanine, or the amino acid corresponding to position 34 of the amino acid sequence of SEQ ID NO: 52 is substituted with alanine, or the amino acid corresponding to position 36 of the amino acid sequence of SEQ ID NO: 53 is substituted with alanine.

Specifically, the protein may consist of any one amino acid sequence of SEQ ID NO: 3 and SEQ ID NO: 57 to SEQ ID NO: 63.

Specifically, the protein may have 55% or more and less than 100% sequence identity to any one amino acid sequence of SEQ ID NO: 3 and SEQ ID NO: 57 to SEQ ID NO: 63.

In one embodiment, the microorganism of the present disclosure may be a microorganism having polyhydroxyalkanoate (PHA)-producing ability.

In any one embodiment of the above-described embodiments, the microorganism of the present disclosure may be a microorganism having 3-hydroxybutyrate-4-hydroxybutyrate copolymer- and/or poly-3-hydroxybutyrate-producing ability.

As used herein, the term "polyhydroxyalkanoate (PHA)" refers to a type of biodegradable bioplastic synthesized by various microorganisms.

As used herein, the term "3-hydroxybutyrate-4-hydroxybutyrate copolymer (poly(3-hydroxybutyrate-co-4-hydroxybutyrate), P(3HB-co-4HB))" is one of polyhydroxyalkanoates (PHAs), and refers to a biodegradable polyester in which 3-hydroxybutyrate and 4-hydroxybutyrate are bound.

As used herein, the term "poly-3-hydroxybutyrate (poly(3-hydroxybutyrate), P(3HB))", which is a polymer of the polyhydroxyalkanoate family, is a polymer of 3-hydroxybutyrate, and is a compound belonging to polyester. The poly-3-hydroxybutyrate may be used interchangeably with poly-3-hydroxybutanoate (P3HA), 3-hydroxybutyrate homopolymer.

As used herein, the term "poly-4-hydroxybutyrate (poly(4-hydroxybutyrate), P(4HB))", which is a polymer of the polyhydroxyalkanoate family, is a polymer of 4-hydroxybutyrate, and is a compound belonging to polyester. The poly-4-hydroxybutyrate may be used interchangeably with poly-4-hydroxybutanoate (P4HA), 4-hydroxybutyrate homopolymer.

As used herein, the term "microorganism (or strain)" comprises all of wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism comprising genetic modification for production of a desired polypeptide, protein, or product. As used herein, the "microorganism", "strain", have the same meaning and may be used interchangeably without limitation.

As used herein, the term "polyhydroxyalkanoate (PHA)-producing microorganism" refers to a prokaryotic or eukaryotic microbial strain capable of producing polyhydroxyalkanoate (PHA) within a living organism, and may comprise both a microorganism prepared by providing the polyhydroxyalkanoate (PHA)-producing ability for a parent strain without the polyhydroxyalkanoate (PHA)-producing ability, or a microorganism that inherently has the polyhydroxyalkanoate (PHA)-producing ability. The polyhydroxyalkanoate (PHA)-producing ability may be conferred or enhanced by species improvement.

In one embodiment, the microorganism of the present disclosure may be a microorganism naturally having the acetoacetyl-CoA reductase variant or the polyhydroxyalkanoate (PHA)-producing ability; or a microorganism prepared by introducing the variant of the present disclosure or the polynucleotide encoding the same (or the vector comprising the polynucleotide) into a parent strain without the acetoacetyl-CoA reductase variant or the polyhydroxyalkanoate (PHA)-producing ability, and/or providing the polyhydroxyalkanoate (PHA)-producing ability therefor, but is not limited thereto.

In one embodiment, the microorganism of the present disclosure may comprise all of a microorganism comprising the sequence of acetoacetyl-CoA reductase variant of the present disclosure due to mutation of the gene encoding the acetoacetyl-CoA reductase variant on the chromosome and/or a microorganism comprising the acetoacetyl-CoA reductase variant of the present disclosure by introducing the vector comprising the polynucleotide encoding the acetoacetyl-CoA reductase variant of the present disclosure, but is not limited thereto.

As used herein, the term "unmodified microorganism" does not exclude strains comprising mutations that may occur naturally in microorganisms, and may be a wild-type strain or a natural strain itself or may be a strain before the trait is changed by genetic variation due to natural or artificial factors. For example, the unmodified microorganism may refer to a strain in which the acetoacetyl-CoA reductase variant described herein is not introduced or a strain before introduction thereof. The "unmodified microorganism" may be used interchangeably with "strain before being modified", "microorganism before being modified", "unvaried strain", "unmodified strain", "unvaried microorganism", or "reference microorganism".

The microorganism having the polyhydroxyalkanoate (PHA)-producing ability of the present disclosure may be a microorganism comprising any one or more of the variant of the present disclosure, the polynucleotide of the present disclosure, and the vector comprising the polynucleotide of the present disclosure; a microorganism modified to express the variant of the present disclosure or the polynucleotide of the present disclosure; a microorganism (e.g., recombinant strain) expressing the variant of the present disclosure or the polynucleotide of the present disclosure; or a microorganism (e.g., recombinant strain) having the activity of the variant of the present disclosure, but is not limited thereto.

For example, the strain of the present disclosure is a cell or microorganism that is transformed with the polynucleotide of the present disclosure or the vector comprising the polynucleotide encoding the variant of the present disclosure to express the variant of the present disclosure. The strain of the present disclosure may comprise all microorganisms capable of producing polyhydroxyalkanoate (PHA) by comprising the variant of the present disclosure. For example, the microorganism of the present disclosure may be a recombinant strain with increased polyhydroxyalkanoate (PHA)-producing ability, in which the acetoacetyl-CoA reductase variant is expressed by introducing the polynucleotide encoding the variant of the present disclosure into a natural wild-type microorganism or a microorganism with the polyhydroxyalkanoate (PHA)-producing ability. The recombinant strain with the increased polyhydroxyalkanoate (PHA)-producing ability may be a microorganism in which the polyhydroxyalkanoate (PHA)-producing ability is increased, as compared to a natural wild-type microorganism or an acetoacetyl-CoA reductase-unmodified microorganism (e.g., a microorganism expressing the wild-type acetoacetyl-CoA reductase or a microorganism not expressing the variant of the present disclosure), but is not limited thereto. For example, the microorganism with the increased polyhydroxyalkanoate (PHA)-producing ability of the present disclosure may be a microorganism in which the polyhydroxyalkanoate (PHA)-producing ability is increased, as compared to a microorganism comprising the polypeptide of SEQ ID NO: 1 or the polynucleotide encoding the same, but is not limited thereto.

The microorganism of the present disclosure may comprise all microorganisms capable of expressing the acetoacetyl-CoA reductase variant of the present disclosure by various known methods in addition to the introduction of the nucleic acid or vector.

For example, the microorganism with the increased polyhydroxyalkanoate (PHA)-producing ability may have an increased polyhydroxyalkanoate (PHA)-producing ability of about 1% or more, specifically, about 1% or more, about 2% or more, about 2.5% or more, about 5% or more, about 10% or more, about 15% or more, about 20% or more, about 25% or more, about 26% or more, about 27% or more, about 28% or more, about 29% or more, about 30% or more, about 31% or more, about 32% or more, or about 33% or more (the upper limit is not particularly limited, but may be, for example, about 200% or less, about 150% or less, about 100% or less, about 50% or less, about 45% or less, or about 40% or less), as compared to that of the parent strain before modification or unmodified microorganism, but the increased amount is not limited thereto as long as the producing ability has an increased amount of a + value, as compared to that of the parent strain before modification or the unmodified microorganism. In another example, the recombinant strain with the increased polyhydroxyalkanoate (PHA)-producing ability may have an increased polyhydroxyalkanoate (PHA)-producing ability of about 1.1 times or more, about 1.12 times or more, about 1.13 times or more, about 1.14 times or more, about 1.15 times or more, about 1.16 times or more, about 1.17 times or more, about 1.18 times or more, about 1.19 times or more, about 1.2 times or more, about 1.21 times or more, about 1.22 times or more, about 1.23 times or more, about 1.24 times or more, about 1.25 times or more, about 1.26 times or more, about 1.27 times or more, about 1.28 times or more, about 1.29 times or more, about 1.30 times or more, about 1.31 times or more, about 1.32 times or more, or about 1.33 times or more (the upper limit is not particularly limited, but may be, for example, about 10 times or less, about 5 times or less, about 3 times or less, about 2 times or less, about 1.5 times or less, about 1.4 times or less), as compared to that of the parent strain before modification or the unmodified microorganism, but is not limited thereto. As used herein, the term "about" refers to a range which comprises all of ±0.5, ±0.4, ±0.3, ±0.2, ±0.1, etc. and comprises all of the values that are equivalent or similar to those following the values, but the range is not limited thereto.

The microorganism of the present disclosure may be a microorganism belonging to *Escherichia* sp., *Erwinia* sp., *Serratia* sp., *Providencia* sp., *Corynebacteria* sp., *Pseudomonas* sp., *Leptospira* sp., *Salmonella* sp., *Brevibacteria* sp., *Hypomononas* sp., *Chromobacterium* sp., and *Norcardia* sp., or fungi, or yeasts, specifically, a microorganism belonging to *Escherichia* sp., and more specifically, *Escherichia coli* (*E. coli*)*,* but is not limited thereto.

Meanwhile, the microorganism belonging to *Escherichia* sp., with the polyhydroxyalkanoate (PHA)-producing ability of the present disclosure may comprise all of a natural wild-type microorganism itself, a microorganism of the *Escherichia* sp. with the improved polyhydroxyalkanoate (PHA)-producing ability by strengthening or weakening the activity of genes related to the polyhydroxyalkanoate (PHA) production mechanism, and a microorganism of the *Escherichia* sp. with the improved polyhydroxyalkanoate (PHA)-producing ability by introducing or strengthening activity of a foreign gene.

Further, the microorganism of the present disclosure may have enhanced acetoacetyl-CoA reductase activity, as compared to the parent strain.

As used herein, the term "enhancement" of the polypeptide activity means that the activity of the polypeptide is increased, as compared to the endogenous activity thereof. The enhancement may be used interchangeably with terms such as activation, up-regulation, overexpression, and increase, etc. Here, the activation, enhancement, up-regulation, overexpression, and increase may comprise both cases in which an activity not originally possessed is exhibited, or the activity is enhanced, as compared to the endogenous activity or the activity before modification. The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification". The fact that the activity of a polypeptide is "enhanced", "up-regulated", "overexpressed", or "increased, as compared to the endogenous activity means that the activity of the polypeptide is improved as compared to the activity and/or concentration (expression level) of the specific polypeptide originally possessed by a parent strain before the trait is changed or an unmodified microorganism.

The enhancement of the polypeptide activity may be achieved by applying a variety methods well known in the art, for example, a method of increasing the intracellular copy number of the gene encoding the variant, a method of introducing a variation into the expression regulatory sequence of the gene encoding the variant on the chromosome, a method of replacing the expression regulatory sequence of the gene encoding the variant on the chromosome by a sequence having strong activity, a method of replacing the gene encoding the protein on the chromosome with a gene which is mutated to increase the activity of the variant, and a method of introducing a variation into the gene encoding the variant protein on the chromosome in order to enhance the activity of the variant, but is not limited thereto.

As used herein, the term "introduction" refers to a method of delivering the polynucleotide encoding the acetohydroxy acid synthase variant or the vector comprising the same to a host cell. Such introduction may be easily performed according to a common method in the art. Generally, there are CaCl₂ precipitation, a Hanahan method that is an improved CaCl₂ method by using dimethyl sulfoxide (DMSO) as a reducing material, electroporation, calcium phosphate precipitation, protoplast fusion, agitation using silicon carbide fiber, PEG-mediated transformation, dextran sulfate-, lipofectamine-, and desiccation/inhibition-mediated transformation, etc. The method of transforming the vector is not limited to the above examples, and transformation or transfection methods commonly used in the art may be used without limitation. Further, as long as the delivered polynucleotide is expressed within the host cell, it may be inserted into and located within the chromosome of the host cell or outside the chromosome. Further, the polynucleotide may be introduced in any form as long as it may be introduced into a host cell and expressed. For example, the polynucleotide may be introduced into a host cell in the form of an expression cassette, which is a polynucleotide construct comprising all elements required for self-expression, but is not limited thereto. The expression cassette may usually comprise a promoter operably linked to an open reading frame (hereinafter, abbreviated to "ORF") of the gene, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

For example, the microorganism of the present disclosure may be a microorganism which has the polyhydroxyalkanoate (PHA)-producing ability by further enhancing the activity of any one of acetyl-CoA acetyltransferase, PHA synthase, and a combination thereof, as compared to the endogenous activity, but is not limited thereto.

For example, the microorganism of the present disclosure may be a microorganism which has the polyhydroxyalkanoate (PHA)-producing ability by further introducing any one of a polynucleotide encoding the acetyl-CoA acetyltransferase or a vector comprising the same; a polynucleotide encoding the PHA synthase or a vector comprising the same; and a combination thereof, but is not limited thereto.

The gene encoding the acetyl-CoA acetyltransferase may be *PhaA* derived from *Cupriavidus necator, Ralstonia eutropha, Ralstonia solanacearum, Zoogloea ramigera, Delftia acidovorans, Bacillus megaterium, Cromobacterium* sp. USM2, *Rhodobacter sphaeroides, Chromobacterium* sp. USM2, *Alcaligenes latus, Azohydromonas lata, Acidovorax species, Burkholderia pseudomallei, Burkholderia vietnamiensis,* or *Burkholderia glumae,* but may comprise any gene without limitation, as long as it is able to express the protein substantially having the acetyl-CoA acetyltransferase activity. A sequence of the *PhaA* gene encoding the acetyl-CoA acetyltransferase may be obtained from NCBI's GenBank or Kyoto Encyclopedia of Genes and Genomes (KEGG), which is a known database. For example, the *PhaA* gene encoding the acetyl-CoA acetyltransferase (SEQ ID NO: 79), derived from *Cupriavidus necator,* may comprise a nucleotide sequence of SEQ ID NO: 80, but is not limited thereto.

The gene encoding the PHA synthase may be *PhaC* derived from *Cupriavidus necator, Ralstonia eutropha, Ralstonia solanacearum, Zoogloea ramigera, Delftia acidovorans, Bacillus megaterium, Cromobacterium* sp. USM2, *Rhodobacter sphaeroides, Chromobacterium* sp. USM2, *Alcaligenes latus, Azohydromonas lata, Acidovorax species, Burkholderia pseudomallei, Burkholderia vietnamiensis,* or *Burkholderia glumae,* but may comprise any gene without limitation, as long as it is able to express the protein substantially having the PHA synthase activity. A sequence of the *PhaC* gene encoding the PHA synthase may be obtained from NCBI's GenBank or Kyoto Encyclopedia of Genes and Genomes (KEGG), which is a known database. For example, the *PhaC* gene encoding the PHA synthase (SEQ ID NO: 81), derived from *Cupriavidus necator,* may comprise a nucleotide sequence of SEQ ID NO: 82, but is not limited thereto.

Still another aspect of the present disclosure provides a method of producing polyhydroxyalkanoate (PHA), the method comprising the step of culturing the microorganism of the present disclosure in a medium.

Specifically, the method of producing polyhydroxyalkanoate (PHA) of the present disclosure may comprise the step of culturing, in a medium, the microorganism comprising the variant of the present disclosure, the polynucleotide of the present disclosure, or the vector of the present disclosure, but is not limited thereto.

As used herein, the term "culturing" refers to growing the microorganism of the present disclosure in appropriately adjusted environment conditions. The culture procedure of the present disclosure may be performed according to appropriate media or culture conditions known in the art. Such culture procedure may be easily adjusted according to the selected strain by a person skilled in the art. Specifically, the culture may be in a batch type, a continuous type, and/or a fed-batch type, but is not limited thereto.

As used herein, the "medium" refers to a mixture comprising, as main ingredients, nutrient materials required for culturing the microorganism of the present disclosure, wherein the medium supplies nutrient materials comprising water essential for survival and growth, growth factors, etc. Specifically, as for the media and other culture conditions used for culturing the microorganism of the present disclosure, any medium that is used for usual culture of microorganisms may be used without particular limitation. However, the microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc.

For example, the culture medium for the strain of *Escherichia* sp. may be found in a literature [Tao H, Bausch C, Richmond C, Blattner FR, Conway T 1999. Functional Genomics: Expression Analysis of Escherichia coli Growing on Minimal and Rich Media. J Bacteriol 181:. Functional Genomics: Expression Analysis of Escherichia coli Growing on Minimal and Rich Media].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

Further, the pH of the culture may be adjusted by adding compounds, such as ammonium hydroxide, potassium hydroxide, ammonia, phosphoric acid, and sulfuric acid, to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-containing gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In the culture of the present disclosure, the culture temperature may be maintained at 20°C to 45°C, specifically, at 25°C to 40°C, and the culture may be performed for about 10 hours to about 160 hours, but is not limited thereto.

The polyhydroxyalkanoate (PHA) produced by the culturing of the present disclosure may be released into the medium or may remain in cells.

The method of producing polyhydroxyalkanoate (PHA) of the present disclosure may further comprise the steps of preparing the microorganism of the present disclosure, preparing a medium for culturing the microorganism, or a combination thereof (regardless of the order, in any order), for example, before the culturing step.

The method of producing polyhydroxyalkanoate (PHA) of the present disclosure may further comprise the step of recovering the polyhydroxyalkanoate (PHA) from a medium resulting from the culturing (a medium in which culturing has been performed) or the microorganism of the present disclosure. The recovering step may be further comprised after the culturing step.

The recovering may be collecting polyhydroxyalkanoate (PHA) by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, etc., HPLC, and a combination of these methods may be used, and polyhydroxyalkanoate (PHA) may be recovered from the medium or microorganism by using an appropriate method known in the art.

In addition, the method of producing polyhydroxyalkanoate (PHA) of the present disclosure may further comprise a purification step. The purification may be performed by using an appropriate method known in the art. In an exemplary embodiment, when the method of producing polyhydroxyalkanoate (PHA) of the present disclosure comprises both the recovering step and the purification step, the recovering step and the purification step may be performed continuously or discontinuously regardless of the order, or may be performed simultaneously or integrated into one step, but is not limited thereto.

Still another aspect of the present disclosure provides a composition for producing polyhydroxyalkanoate (PHA), the composition comprising the protein; the polynucleotide encoding the protein; the vector comprising the polynucleotide; or the microorganism comprising the protein, the polynucleotide encoding the protein, or the vector comprising the polynucleotide; a culture of the microorganism; or a combination of two or more thereof.

The composition of the present disclosure may further comprise any appropriate excipient that is usually used in the composition for producing amino acids, and such excipients may comprise, for example, a preserving agent, a wetting agent, a dispersing agent, a suspending agent, a buffering agent, a stabilizing agent, an isotonic agent, etc., but are not limited thereto.

In a specific embodiment, each component present in the composition of the present disclosure may be comprised in a microbiologically effective amount, or in an amount to be appropriately present in the composition for production.

Still another aspect of the present disclosure provides use of the protein having acetoacetyl-CoA reductase activity, in which an amino acid at position 35 of the amino acid sequence of SEQ ID NO: 1 or an amino acid sequence having 55% or more sequence identity to the amino acid sequence of SEQ ID NO: 1 is substituted with a different amino acid; or the microorganism comprising the protein, the polynucleotide encoding the protein, or the vector comprising the polynucleotide in producing polyhydroxyalkanoate (PHA).

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Preparation of a strain expressing acetoacetyl-CoA reductase variant and Confirmation of P(3HB) production capability - 1

### Example 1-1. Vector construction for preparing a P(3HB)-producing strain

To prepare P(3HB)-producing *Escherichia coli,* a vector comprising *phaA, phaB,* and *phaC* was constructed.

In detail, a pCL1920 vector (GenBank No AB236930) was used to construct a pCL-PuspA_phaA-PuspA_phaC-PuspA_phaB vector comprising a PuspA promoter (universal stress protein A promoter) (Prytz et al. 2003; Dyk et al. 1995; Nystrϕm and Neidhardt 1992; 1994) to express *Cupriavidus necator*-derived *phaA* (SEQ ID NO: 80), *phaC* (SEQ ID NO: 82), and *phaB* (SEQ ID NO: 2) gene sequences in *Escherichia coli.* PCR was performed using primers of SEQ ID NOS: 4 and 5, based on a reference sequence NZ_CP039287.1 of the NCBI database, to amplify the *phaA, phaC,* and *phaB* genes.

PCR was performed with 30 cycles of denaturation at 95°C for 30 seconds, annealing at 55°C for 30 seconds, and extension at 72°C for 1 minute. This PCR product was electrophoresed on a 1.0% agarose gel, and then the band was purified by elution, and the pCL1920 vector (GenBank No AB236930) was treated with Xbal and Pstl restriction enzymes. The restriction enzyme-treated pCL1920 vector and the insert DNA fragment amplified through the above PCR were linked using an Infusion Cloning Kit, then transformed into *E. coli* DH5α.Colonies were selected on an LB medium comprising 75 mg/L of spectinomycin antibiotic.

### Example 1-2. Vector construction for preparing a P(3HB)-producing strain expressing acetoacetyl-CoA reductase variant

A vector for expressing the acetoacetyl-CoA reductase variant in a strain was constructed. In detail, PCR was performed under the same conditions as Example 1-1, based on a reference sequence NZ_CP039287.1 in the NCBI database, using SEQ ID NOS: 6, 7 and 4, 5 for introduction of M12S mutation into *phaB,* SEQ ID NOS: 8, 9 and 4, 5 for introduction of G35A mutation, and SEQ ID NOS: 10, 11 and 4, 5 for introduction of N61D mutation, SEQ ID NOS: 12, 13 and 4, 5 for introduction of V62T mutation, SEQ ID NOS: 14, 15 and 4, 5 for introduction of F148Y mutation, SEQ ID NOS: 16, 17 and 4, 5 for introduction of I194M mutation, SEQ ID NOS: 18, 19 and 4, 5 for introduction of V198T mutation, SEQ ID NOS: 20, 21 and 4, 5 for introduction of E47L mutation, and SEQ ID NOS: 22, 23 and 4, 5 for introduction of T173S mutation. Primer sequence information is shown in Table 1.

**[Table 1]**

| Primer | Nucleotide sequence |
|---|---|
| SEQ ID NO: 4 | 5’- CGGTACCCGGGGATCCTCTAGAGAACCACTATCAATATATTCA TGTCGAA |
| SEQ ID NO: 5 | 5'-CAAGCTTGCATGCCTGCAGGTTTGCCTGGCGGCAGTA |
| SEQ ID NO: 6 | 5'-gcgtatgtgaccggcggcAGCggtgtatcggaaccgcc |
| SEQ ID NO: 7 | 5'-ggcggttccgataccaccGCTgccgccggtcacatacgc |
| SEQ ID NO: 8 | 5'-cgtgtggtggccggttgcGCCcccaactcgccgcgccgcg |
| SEQ ID NO: 9 | 5'-cgcggcgcggcgagttgggGGCgcaaccggccaccacacg |
| SEQ ID NO: 10 | 5'-gatttcattgcctcggaaggcGATGTGgctgactgggactcgaccaagac |
| SEQ ID NO: 11 | 5'-gtcttggtcaagtcccagtcagcCACATCaccttccgagacaatgaaatc |
| SEQ ID NO: 12 | 5'-gatttcattgcctcggaagacAATACGgctgactgggactcgaccaag |
| SEQ ID NO: 13 | 5'-cttggtcgagtcccagtcagcCGTATTgccttccggaggcaatgaaatc |
| SEQ ID NO: 14 | 5'-gaacgggcagaagggccagTACggccagaccaactactccac |
| SEQ ID NO: 15 | 5'-gtggagtagttgatctggccGTActggacccttctgcccgttc |
| SEQ ID NO: 16 | 5'-ccaccgacatggtcaaggcgATGcgccaggacGTGctcgac |
| SEQ ID NO: 17 | 5'-gtcgagCACgtcctggcgCATcgccttgaccatgtcggtgg |
| SEQ ID NO: 18 | 5'-ggtcaaggcgATCcgccaggacACGctcgacaagatcgtcgcg |
| SEQ ID NO: 19 | 5'-cgcgacgatcttgtcgagCGTgtcctggcgGATcgccttgacc |
| SEQ ID NO: 20 | 5'-cgcgaaaagtggctggagCTGcagaaggccctgggcttcg |
| SEQ ID NO: 21 | 5'-cgaagcccaggggccttctgCAGctccagccacttttcgcg |
| SEQ ID NO: 22 | 5'-ctggcgcaggaagtggcgTCCaaggacatgaccgtcaacac |
| SEQ ID NO: 23 | 5'-gtgttgacggtcacgcccttGGAcgccacttcctgcgccag |

Expression vectors for *phaA, phaC,* and the respective *phaB* mutant genes obtained from cloning results were named pCL-PuspA_phaA-PuspA_phaC-PuspA_phaB(M12S), pCL-PuspA_phaA-PuspA_phaC-PuspA_phaB(G35A), pCL-PuspA_phaA-PuspA_phaC-PuspA_phaB(N61D), pCL-PuspA_phaA-PuspA_phaC-PuspA_phaB(V62T), pCL-PuspA_phaA-PuspA_phaC-PuspA_phaB (F148Y), pCL-PuspA_phaA-PuspA_phaC-PuspA_phaB(I194M), pCL-PuspA_phaA-PuspA_phaC-PuspA_phaB(V198T), pCL-PuspA_phaA-PuspA_phaC-PuspA_phaB(Q47L), and pCL-PuspA_phaA-PuspA_phaC-PuspA_phaB(T173S), respectively.

### Example 1-3. Preparation of a strain expressing acetoacetyl-CoA reductase variant and Confirmation of P(3HB) production capability

To evaluate the P(3HB) production capability according to the expression of the acetoacetyl-CoA reductase variant, *E.coli* strains were constructed by introducing the wild-type *phaB* and *phaB* mutation respectively. Specifically, each of the plasmids prepared in Examples 1-1 and 1-2 was introduced into wild-type *E. coli* by the TSS method, and the strains were selected on an LB medium comprising 75 mg/L of spectinomycin antibiotic (Proc Natl Acad Sci U S A. 1989 Apr; 86(7): 2172-5).

Subsequently, a P(3HB) production capability test was performed on the selected strains. Each strain was inoculated into a 250 mL corner-baffled flask comprising 25 mL of P(3HB) production medium comprising 5% glucose, and then cultured with shaking at 230 rpm for 5 hours at 37°C and 43 hours at 35°C. Thereafter, P(3HB) concentrations were analyzed by gas chromatography. The analyzed P(3HB) concentrations are shown in Table 2 below.

**[Table 2]**

| Strain | P(3HB) concentration (mg) | Yield (%) |
|---|---|---|
| E.coli / pCL-PuspA_phaA-PuspA_phaC-PuspA_phaB | 11.2 | 22.4 |
| E.coli / pCL-PuspA_phaA-PuspA_phaC-PuspA_phaB (M12S) | 7.5 | 15.0 |
| E.coli / pCL-PuspA_phaA-PuspA_phaC-PuspA_phaB (G35A) | 13.2 | 26.4 |
| E.coli / pCL-PuspA_phaA-PuspA_phaC-PuspA_phaB (N61D) | 11.9 | 23.8 |
| E.coli / pCL-PuspA_phaA-PuspA_phaC-PuspA_phaB (V62T) | 8.9 | 17.8 |
| E.coli / pCL-PuspA_phaA-PuspA_phaC-PuspA_phaB (F148Y) | 11.6 | 23.1 |
| E.coli / pCL-PuspA_phaA-PuspA_phaC-PuspA_phaB (I194M) | 12.5 | 25.1 |
| E.coli / pCL-PuspA_phaA-PuspA_phaC-PuspA_phaB (V198T) | 11.1 | 22.2 |
| E.coli / pCL-PuspA_phaA-PuspA_phaC-PuspA_phaB (Q47L) | 10.9 | 21.8 |
| E.coli / pCL-PuspA_phaA-PuspA_phaC-PuspA_phaB (T173S) | 11.9 | 23.8 |

As a result, the P(3HB) production capability of the strain expressing acetoacetyl-CoA reductase G35A variant increased by 4%, as compared to that of - the strain expressing the wild-type *phaB,* confirming that the position 35 of an amino acid sequence is an important position for P(3HB) production.

### Example 2. Residue mutation experiment of acetoacetyl-CoA reductase variant (G35)

### Example 2-1. Vector construction

In Example 1, it was confirmed that the position 35 of an amino acid sequence of acetoacetyl-CoA reductase is an important position for polyhydroxyalkanoate (PHA) production, and therefore, a mutant strain was prepared based on the wild-type *E. coli* to express a mutant protein in which the amino acid at position 35 of an amino acid sequence of acetoacetyl-CoA reductase was substituted with a different amino acid, to determine whether or not the P(3HB) production capability was increased.

In detail, PCR was performed in the same manner as Example 1-1 for introduction of *phaA, phaC,* and *phaB* variant coding sequence encoding acetoacetyl-CoA reductase, by using SEQ ID NOS: 24, 25 and 4, 5 for introduction of G35H variant, SEQ ID NOS: 26, 27 and 4, 5 for introduction of G35V variant coding sequence, and SEQ ID NO: 28, 29 and 4, 5 for introduction of G35S variant coding sequence. Primer sequence information is shown in Table 3.

**[Table 3]**

| Primer | Nucleotide sequence |
|---|---|
| SEQ ID NO: 24 | 5'-cgtgtggtggccggttgcCACcccaactcgccgcgccgcg |
| SEQ ID NO: 25 | 5'-cgcggcgcggcgagttgggGTGgcaaccaggcaccacacg |
| SEQ ID NO: 26 | 5'-cgtgtggtggccggttgcGTCcccaactcgccgcgccgcg |
| SEQ ID NO: 27 | 5'-cgcggcgcggcgagttgggGACgcaaccggccaccacacg |
| SEQ ID NO: 28 | 5'-cgtgtggtggccggttgcTCCcccaactcgccgcgccgcg |
| SEQ ID NO: 29 | 5'-cgcggcgcggcgagttgggGGAgcaaccggccaccacacg |

Cloning was performed in the same manner as Example 1, and the *phaB* mutation-introduced vectors obtained from cloning results were named pCL-PuspA_phaA-PuspA_phaC-PuspA_phaB(G35H), pCL-PuspA_phaA-PuspA_phaC-PuspA_phaB(G35V), and pCL-PuspA_phaA-PuspA_phaC-PuspA_phaB(G35S), respectively.

### Example 2-2. Preparation of a strain expessing acetoacetyl-CoA reductase variant and Confirmation of P(3HB) production capability

To confirm the P(3HB) production capability of the strain expressing the acetoacetyl-CoA reductase variant, *phaB* comprising mutations and *phaA, phaC* were expressed together based on wild-type *E. coli.* Vectors comprising the *phaB* mutations prepared in Example 2-1 (pCL-PuspA_phaA-PuspA_phaC-PuspA_phaB(G35H), pCL-PuspA_phaA-PuspA_phaC-PuspA_phaB(G35V), and pCL-PuspA_phaA-PuspA_phaC-PuspA_phaB (G35S)) were each introduced into wild-type *E. coli* by the TSS method and strains were selected on an LB medium comprising 75 mg/L of spectinomycin antibiotic (Proc Natl Acad Sci U S A. 1989 Apr; 86(7): 2172-5).

Subsequently, a P(3HB) production capability test was performed on the selected strains. Each strain was inoculated into a 250 mL corner-baffled flask comprising 25 mL of P(3HB) production medium comprising 5% glucose, and then cultured with shaking at 230 rpm for 5 hours at 37°C and 43 hours at 35°C. Thereafter, P(3HB) concentrations were analyzed by gas chromatography. The analyzed P(3HB) concentrations are shown in Table 4 below.

**[Table 4]**

| Strain | P(3HB) concentration (mg) | Yield (%) |
|---|---|---|
| E.coli / pCL-PuspA_phaA-PuspA_phaC-PuspA_phaB | 11.1 | 22.2 |
| E.coli / pCL-PuspA_phaA-PuspA_phaC-PuspA_phaB(G35A) | 13.3 | 26.6 |
| E.coli / pCL-PuspA_phaA-PuspA_phaC-PuspA_phaB(G35H) | 11.0 | 22.0 |
| E.coli / pCL-PuspA_phaA-PuspA_phaC-PuspA_phaB(G35V) | 7.2 | 14.3 |
| E.coli / pCL-PuspA_phaA-PuspA_phaC-PuspA_phaB(G35S) | 10.1 | 20.2 |

As a result, it was confirmed that the variant in which glycine at position 35 of acetoacetyl-CoA reductase was substituted with alanine significantly increased the P(3HB) production capability. In contrast, it was confirmed that when glycine at position 35 of acetoacetyl-CoA reductase was substituted with histidine, valine, or serine, respectively, the P(3HB) yield was equal to or less than that of the wild-type phaB-expressing strain, suggesting that it is significant in increasing the P(3HB) production capability only when the amino acid at position 35 is substituted with alanine.

### Example 3. Preparation of a strain expressing acetoacetyl-CoA reductase variant and Confirmation of P(3HB) production capability - 2

### Example 3-1. Vector construction

With respect to the *phaB* genes derived from other species, in addition to the *phaB* gene derived from *Cupriavidus necator* which was identified in Example 1, the effects of mutations in which the amino acid at the position corresponding to position 35 of an amino acid sequence of *phaB* derived from *Cupriavidus necator* of Example 1-1 was substituted with alanine were examined. In detail, with respect to each of *phaB* genes derived from 7 types of strains (*Pseudomonas putida, Alcaligenes latus, Allochromatium vinosum* DSM 180, *Azotobacter beijerinckii, Pandoraea* sp. B-6, *Burkholderiaceae bacterium* 16, *Candidatus Accumulibacter phosphatis*) which are structurally similar to each other while having 55% or more homology to the *phaB* gene encoding acetoacetyl-CoA reductase derived from *Cupriavidus necator* of Example 1, *phaB* genes encoding acetoacetyl-CoA reductase variants (SEQ ID NO: 57 to SEQ ID NO: 63, respectively), each comprising the mutation in which the amino acid corresponding to position 35 of an amino acid sequence of *Cupriavidus necator-*derived *phaB* of Example 1-1 was substituted with alanine, were amplified in order to express the gene in *Escherichia coli.* Each gene comprising the mutation was subjected to PCR (Appl Environ Microbiol. 2013 Oct;79(19):6134-9), and primer sequence information is shown in Table 5.

**[Table 5]**

| Origin | Primer | Nucleotide sequence |
|---|---|---|
| *Pseudomonas putida* | SEQ ID NO: 30 | 5’- CGGTCGTCGCGGGTTGCGcCCCGAACT CTCCGCGCC |
| | SEQ ID NO: 31 | 5’- GGCGCGGAGAGTTCGGGgCGCAACCCG CGACGACCG |
| *Allochromatium vinosum* DSM 180 | SEQ ID NO: 32 | 5’- CTGCACCGTCGTGGCGAACTGCgcTCCG TCCGAGGCGGCC |
| | SEQ ID NO: 33 | 5’- GGCCGCCTCGGACGGAgcGCAGTTCGC CACGACGGTGCAG |
| *Azotobacter beijerinckii* | SEQ ID NO: 34 | 5’- CAAAGTCATCGTCGGATGCgCCCCACAA TCCAAACGCAAAG |
| | SEQ ID NO: 35 | 5’- CTTTGCGTTTGGATTGTGGGGcGCATCC GACGATGACTTTG |
| *Alcaligenes latus* | SEQ ID NO: 36 | 5’- GGTCATCGCCGGCTGCGcTCCGAGCCG CGACTACCAGAAG |
| | SEQ ID NO: 37 | 5’- CTTCTGGTAGTCGCGGCTCGGAgCGCA GCCGGCGATGACC |
| *Pandoraea* sp. B-6 | SEQ ID NO: 38 | 5’- ccgcgttgcagtgacctgtGcccccacgaatcacgattg |
| | SEQ ID NO: 39 | 5’- caatcgtgattcgtgggggCacaggtcactgcaacgcgg |
| *Burkholderiaceae bacterium 16* | SEQ ID NO: 40 | 5’- CTACGCGGTCGTCGTGACGCATgCGCC GGGCAACGCGAAGG |
| | SEQ ID NO: 41 | 5’- CCTTCGCGTTGCCCGGCGcATGCGTCAC GACGACCGCGTAG |
| *Candidatus Accumulibacter phosphatis* | SEQ ID NO: 42 | 5’- CACAAGGTGGTTGCCGCCTACgcTCCGG AGTTCGACAAGC |
| | SEQ ID NO: 43 | 5’- GCTTGTCGAACTCCGGAgcGTAGGCGGC AACCACCTTGTG |

PCR was performed with 30 cycles of denaturation at 95°C for 30 seconds, annealing at 55°C for 30 seconds, and extension at 72°C for 1 minute. This PCR product was electrophoresed on a 1.0% agarose gel, and then the band was purified by elution, and the pCL1920 vector was treated with EcoRV and Hindlll restriction enzymes. The restriction enzyme-treated pCL1920 vector and the insert DNA fragment amplified through the above PCR were linked using an Infusion Cloning Kit, then transformed into *E. coli* DH5α, and colonies were selected on an LB medium comprising 75 mg/L of spectinomycin antibiotic. The vectors comprising the *phaA* and *phaC* genes, and foreign species (*Pseudomonas putida, Alcaligenes latus, Allochromatium vinosum* DSM 180, *Azotobacter beijerinckii, Pandoraea* sp. B-6, *Burkholderiaceae bacterium* 16, *Candidatus Accumulibacter phosphatis*)-derived phaB into which mutations were introduced, which were obtained from cloning result, were named pCL-PuspA_phaA-PuspA_phaC-PuspA_Ppu_phaB (G35A), pCL-PuspA_phaA-PuspA_phaC-PuspA__Aau_phaB (G35A), pCL-PuspA_phaA-PuspA_phaC-PuspA_Avi_phaB (H34A), pCL-PuspA_phaA-PuspA_phaC-PuspA_Abe_phaB (T36A), pCL-PuspA_phaA-PuspA_phaC-PuspA_P_phaB (S35A), pCL-PuspA_phaA-PuspA_phaC_PuspA_Bba_phaB (T35A), and pCL-PuspA_phaA_PuspA_phaC_PuspA_Cac_phaB (H35A), respectively.

### Example 3-2. Preparation of a strain expressing acetoacetyl-CoA reductase variant and Confirmation of P(3HB) production capability

The *phaA, phaC* and *phaB* comprising mutations were expressed in the wild-type *E. coli,* as in Example 1-2. The vectors (pCL-PuspA_phaA-PuspA_phaC-PuspA_Ppu_phaB (G35A), pCL-PuspA_phaA-PuspA_phaC-PuspA_Aau_phaB (G35A), pCL-PuspA_phaA-PuspA_phaC-PuspA_Avi_phaB (H34A), pCL-PuspA_phaA-PuspA_phaC-PuspA_Abe_phaB (T36A), pCL-PuspA_phaA-PuspA_phaC-PuspA_P_phaB (S35A), pCL-PuspA_phaA-PuspA_phaC-PuspA_Bba_phaB (T35A), and pCL-PuspA_phaA-PuspA_phaC-PuspA_Cac_phaB (H35A)) comprising the *phaB* mutations derived from each origin, which were prepared in Example 3-1, were each introduced into the wild-type *E. coli* by the TSS method and stains were selected on an LB medium comprising 75 mg/L of spectinomycin antibiotic (Proc Natl Acad Sci U S A. 1989 Apr; 86(7): 2172-5).

Subsequently, a P(3HB) production capability test was performed on the selected strains. Each strain was inoculated into a 250 mL corner-baffled flask comprising 25 mL of P(3HB) production medium comprising 5% glucose, and then cultured with shaking at 230 rpm for 5 hours at 37°C and 43 hours at 35°C. P(3HB) concentrations were analyzed by gas chromatography. The test results are shown in Table 6 below.

**[Table 6]**

| Strain | P(3HB) concentration (mg) | Yield (%) |
|---|---|---|
| E.coli / pCL-PuspA_phaA-PuspA_phaC-PuspA_Ppu_phaB | 10.4 | 20.8 |
| E.coli / pCL-PuspA_phaA-PuspA_phaC-PuspA_Ppu_phaB (G35A) | 12.6 | 25.2 |
| E.coli / pCL-PuspA_phaA-PuspA_phaC-PuspA_Aau_phaB | 10.2 | 20.4 |
| E.coli / pCL-PuspA_phaA-PuspA_phaC-PuspA_Aau_phaB (G35A) | 12.0 | 24 |
| E.coli / pCL-PuspA_phaA-PuspA_phaC-PuspA_Avi_phaB | 5.2 | 10.4 |
| E.coli / pCL-PuspA_phaA-PuspA_phaC-PuspA_Avi_phaB (H34A) | 6.6 | 13.2 |
| E.coli / pCL-PuspA_phaA-PuspA_phaC-PuspA_Abe_phaB | 6.1 | 12.2 |
| E.coli / pCL-PuspA_phaA-PuspA_phaC-PuspA_Abe_phaB (T36A) | 7.3 | 14.6 |
| E.coli / pCL-PuspA_phaA-PuspA_phaC-PuspA_P_phaB | 5.9 | 11.8 |
| E.coli / pCL-PuspA_phaA-PuspA_phaC-PuspA_P_phaB (S35A) | 7.0 | 14 |
| E.coli / pCL-PuspA_phaA-PuspA_phaC-PuspA_Bba_phaB | 4.5 | 9 |
| E.coli / pCL-PuspA_phaA-PuspA_phaC-PuspA_Bba_phaB (T35A) | 6.0 | 12 |
| E.coli / pCL-PuspA_phaA-PuspA_phaC-PuspA_Cac_phaB | 5.1 | 10.2 |
| E.coli / pCL-PuspA_phaA-PuspA_phaC-PuspA_Cac_phaB (H35A) | 6.1 | 12.2 |

As a result, as shown in Table 6, it was confirmed that when the strains each expressing the *phaB* comprising the amino acid mutation derived from each foreign species were cultured, the P(3HB) yield was increased, as compared to that of the wild-type *phaB* expression. Therefore, when the amino acid at position corresponding to the amino acid at position 35 of an amino acid sequence of acetoacetyl-CoA reductase expressed by *phaB* derived from *Cupriavidus necator* was substituted with alanine in the *phaB* derived from various foreign species, the activity of the enzyme expressed by *phaB* was enhanced thereby increasing the P(3HB) production capability.

### Example 4. Preparation of a strain expressing acetoacetyl-CoA reductase variant and Confirmation of P(3HB-co-4HB) production capability

### Example 4-1. Vector construction

In order to construct a vector capable of inserting the acetoacetyl-CoA reductase G35A mutation coding sequence *phaB*(G35A) into the genome, a pSKH vector (KR 10-2006-0137650 A) was used. In detail, PCR was performed using pCL-PuspA_phaA-PuspA_phaC-PuspA_phaB(G35A) prepared in Example 1-1 as a template and SEQ ID NOS: 44 and 45. To comprise a region with the same sequence as the desired insertion position, PCR was performed using SEQ ID NOS: 46, 47 and 48, 49, based on genomic DNA of *E*. *coli.* Primer sequence information is shown in Table 7.

**[Table 7]**

| Primer | Nucleotide sequence |
|---|---|
| SEQ ID NO: 44 | 5'- CTCACGATGCTCGCGTATTGAgatatcAACCACTATCAAT ATATTCATG |
| SEQ ID NO: 45 | 5’- CGAAAGTTTGAGGCGTAAAAAGCGATATCTTTGCCTGG CGGCAGTAG |
| SEQ ID NO: 46 | 5’- CCGGGCTGCAGGAATTCGATACGAAACACGCGTTCATT GA |
| SEQ ID NO: 47 | 5'- CATGAATATATTGATAGTGGTTgatatcTCAATACGCGAG CATCGTGAG |
| SEQ ID NO: 48 | 5’- CTACTGCCGCCAGGCAAAGATATCGCTTTTTACGCCTC AAACTTTCG |
| SEQ ID NO: 49 | 5’- GCTAGGTCGACTAGCGTGATGCACACGCCGATGCTTTT GA |

PCR was performed with 30 cycles of denaturation at 95°C for 30 seconds, annealing at 55°C for 30 seconds, and extension at 72°C for 1 minute. This PCR product was electrophoresed on a 1.0% agarose gel, and then the band was purified by elution, and the pSKH vector was treated with EcoRV restriction enzyme. The restriction enzyme-treated pSKH vector and the insert DNA fragment amplified through the above PCR were linked using an Infusion Cloning Kit, then transformed into *E. coli* DH5α, and colonies were selected on an LB medium comprising 50 mg/L of kanamycin antibiotic. The phaB gene-expressing vector obtained from cloning result was named pSKH-PuspA-phaB(G35A).

### Example 4-2. Preparation of a strain expressing acetoacetyl-CoA reductase variant and Confirmation of P(3HB-co-4HB) production capability

To examine changes in the ratio of 3HB and 4HB through the 3HB increase according to the introduction of the variant *phaB*(G35A) gene based on the P(3HB-co-4HB) producing strain, a P(3HB-co-4HB)-producing strain (Strain 13) previously prepared was used (US 10323261 B1). Based on the P(3HB-co-4HB)-producing strain (Strain 13), pSKH-PuspA-phaB(G35A) prepared in Example 3-1 was transformed by the electric pulse method. The prepared strain was named *Escherichia coli* CB02-6589, and deposited under the Budapest Treaty in the international depository authority, Korean Culture Center of Microorganisms (KCCM), on December 09, 2022, with the Accession No. KCCM13301P.

Subsequently, the P(3HB-co-4HB) production capability of the P(3HB-co-4HB)-producing strain CB02-6589 into which the variant *phaB*(G35A) was introduced was tested. The analyzed concentration of P(3HB-co-4HB) and the content of 4HB are shown in Table 6 below.

**[Table 8]**

| Strain | P(3HB-co-4HB) concentration (mg) | 3HB content (%) | Yield (%) |
|---|---|---|---|
| Strain 13 | 12.8 | 81.5 | 25.6 |
| CB02-6589 | 14.5 | 89.5 | 29.0 |

As a result, it was confirmed that the strain expressing acetoacetyl-CoA reductase variant (G35A) showed 3HB increase due to enhancement of the 3HB biosynthetic pathway, as compared to the parent strain, and the 3HB content increased by about 8%. It was also confirmed that the P(3HB-co-4HB) yield increased by 4.6% due to enhancement of the acetoacetyl-CoA reductase enzyme activity, as compared to that of the parent strain.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

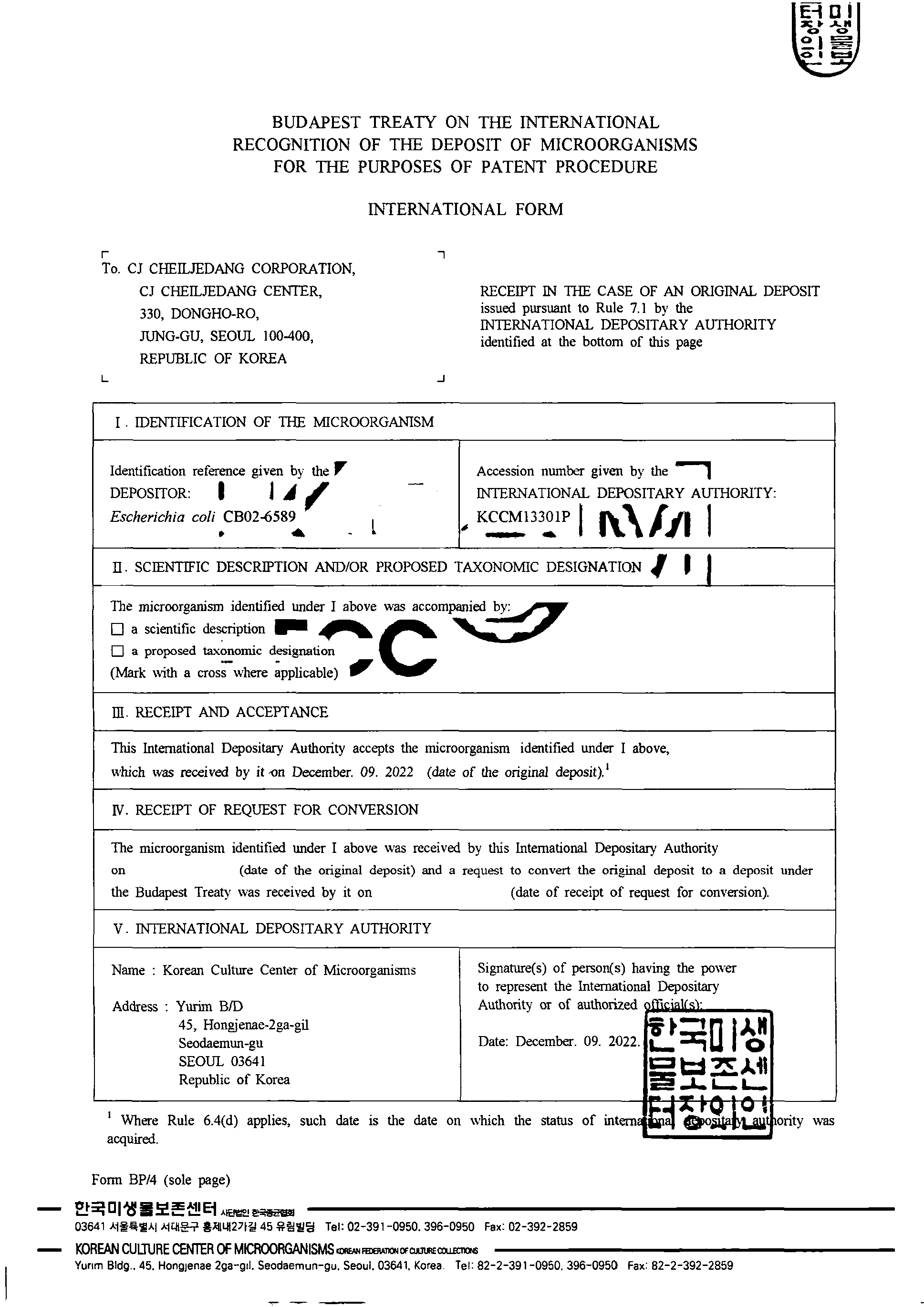
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Escherichia coli CB02-6589 | Accession number given by the
INTERNATIONAL DEPOSITARY AUTHORITY:
KCCM13301P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on December. 09. 2022 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 09. 2022.


¹ Where Rule 6.4(d) applies, such date is the date on which the status of international depositary authority was
acquired.

Form BP/4 (sole page)

한국미생물보존센터 사단법인 한국종균협회
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

## Claims

1. A protein having acetoacetyl-CoA reductase activity, wherein an amino acid corresponding to position 35 of an amino acid sequence of SEQ ID NO: 1 or an amino acid sequence having 55% or more sequence identity to the amino acid sequence of SEQ ID NO: 1 is substituted with a different amino acid.

2. The protein of claim 1, wherein an amino acid corresponding to position 35 of any one amino acid sequence of SEQ ID NO: 50, SEQ ID NO: 51, and SEQ ID NO: 54 to SEQ ID NO: 56 is substituted with a different amino acid,
an amino acid corresponding to position 34 of an amino acid sequence of SEQ ID NO: 52 is substituted with a different amino acid, or
an amino acid corresponding to position 36 of an amino acid sequence of SEQ ID NO: 53 is substituted with a different amino acid.

3. The protein of claim 1, wherein an amino acid corresponding to position 35 of the amino acid sequence of SEQ ID NO: 1 is substituted with alanine,
an amino acid corresponding to position 35 of any one amino acid sequence of SEQ ID NO: 50, SEQ ID NO: 51, and SEQ ID NO: 54 to SEQ ID NO: 56 is substituted with alanine,
an amino acid corresponding to position 34 of the amino acid sequence of SEQ ID NO: 52 is substituted with alanine, or
an amino acid corresponding to position 36 of the amino acid sequence of SEQ ID NO: 53 is substituted with alanine.

4. The protein of claim 1, wherein the protein consists of any one amino acid sequence of SEQ ID NO: 3 and SEQ ID NO: 57 to SEQ ID NO: 63.

5. The protein of claim 4, wherein the protein has an amino acid sequence having 55% or more and less than 100% sequence identity to any one amino acid sequence of SEQ ID NO: 3 and SEQ ID NO: 57 to SEQ ID NO: 63.

6. A polynucleotide encoding the protein of any one of claims 1 to 5.

7. A microorganism comprising a protein having acetoacetyl-CoA reductase activity, wherein an amino acid corresponding to position 35 of an amino acid sequence of SEQ ID NO: 1 or an amino acid sequence having 55% or more sequence identity to the amino acid sequence of SEQ ID NO: 1 is substituted with a different amino acid, a polynucleotide encoding the protein, or a vector comprising the polynucleotide.

8. The microorganism of claim 7, wherein the protein is one in which an amino acid corresponding to position 35 of any one amino acid sequence of SEQ ID NO: 50, SEQ ID NO: 51, and SEQ ID NO: 54 to SEQ ID NO: 56 is substituted with a different amino acid,
an amino acid corresponding to position 34 of an amino acid sequence of SEQ ID NO: 52 is substituted with a different amino acid, or
an amino acid corresponding to position 36 of an amino acid sequence of SEQ ID NO: 53 is substituted with a different amino acid.

9. The microorganism of claim 7, wherein the protein is one in which an amino acid corresponding to position 35 of the amino acid sequence of SEQ ID NO: 1 is substituted with alanine,
an amino acid corresponding to position 35 of any one amino acid sequence of SEQ ID NO: 50, SEQ ID NO: 51, and SEQ ID NO: 54 to SEQ ID NO: 56 is substituted with alanine,
an amino acid corresponding to position 34 of the amino acid sequence of SEQ ID NO: 52 is substituted with alanine, or
an amino acid corresponding to position 36 of the amino acid sequence of SEQ ID NO: 53 is substituted with alanine.

10. The microorganism of claim 7, wherein the protein consists of any one amino acid sequence of SEQ ID NO: 3 and SEQ ID NO: 57 to SEQ ID NO: 63.

11. The microorganism of claim 7, wherein the protein has 55% or more and less than 100% sequence identity to any one amino acid sequence of SEQ ID NO: 3 and SEQ ID NO: 57 to SEQ ID NO: 63.

12. The microorganism of claim 7, wherein the microorganism has increased polyhydroxyalkanoate (PHA)-producing ability, as compared to a microorganism comprising any one polypeptide of SEQ ID NO: 1 and SEQ ID NO: 50 to SEQ ID NO: 56 or a polynucleotide encoding the same.

13. The microorganism of claim 7, wherein the microorganism is a microorganism of *Escherichia* sp.

14. The microorganism of claim 13, wherein the microorganism of *Escherichia* sp. is *Escherichia coli.*

15. A method of producing polyhydroxyalkanoate (PHA), the method comprising the step of culturing the microorganism of claim 7 in a medium.

16. The method of claim 15, further comprising the step of recovering a desired material from the cultured microorganism, a culture of the microorganism, a fermentation product of the microorganism, or the culture medium.

17. A composition for producing polyhydroxyalkanoate (PHA), the composition comprising:
a protein having acetoacetyl-CoA reductase activity, wherein an amino acid corresponding to position 35 of an amino acid sequence of SEQ ID NO: 1 or an amino acid sequence having 55% or more sequence identity to the amino acid sequence of SEQ ID NO: 1 is substituted with a different amino acid;
a polynucleotide encoding the protein;
a microorganism comprising the protein, the polynucleotide encoding the protein, or a vector comprising the polynucleotide;
a culture of the microorganism; or
a combination of two or more thereof.

18. Use of a protein having acetoacetyl-CoA reductase activity, wherein an amino acid corresponding to position 35 of an amino acid sequence of SEQ ID NO: 1 or an amino acid sequence having 55% or more sequence identity to the amino acid sequence of SEQ ID NO: 1 is substituted with a different amino acid; or
a microorganism comprising the protein, a polynucleotide encoding the protein, or a vector comprising the polynucleotide, for producing polyhydroxyalkanoate (PHA).
